# EUROPÄISCHE PATENTSCHRIFT

(11) **EP 1 748 808 B1**
(45) Veröffentlichungstag und Bekanntmachung des Hinweises auf die Patenterteilung: **02.01.2013**
(21) Anmeldenummer: 05739700.2
(22) Anmeldetag: 10.05.2005
(51) Int. Cl.: A61M 1/10, A61N 1/362

(54) **VORRICHTUNG ZUR EPIKARDIALEN UNTERSTÜTZUNG UND/ODER ÜBERNAHME DER HERZTÄTIGKEIT**
DEVICE FOR EPICARDIAL SUPPORT AND/OR RESUMPTION OF CARDIAC ACTIVITY
DISPOSITIF D'ASSISTANCE EPICARDIQUE ET/OU REPRISE DE L'ACTIVITE CARDIAQUE

(30) Priorität: 11.05.2004 DE 102004023190
(43) Veröffentlichungstag der Anmeldung: 07.02.2007
(73) Patentinhaber: PPA Technologies AG, 07745 Jena (DE)
(72) Erfinder: FERRARI, Markus, 07747 Jena (DE)
(74) Vertreter: Weidener, Jörg Michael
(86) Internationale Anmeldenummer: PCT/EP2005/005051
(87) Internationale Veröffentlichungsnummer: WO 2005/110512

(56) Entgegenhaltungen:
- DE-A1- 19 951 220
- US-A1- 2001 041 821
- US-A1- 2003 088 151
- US-A1- 2003 105 481
- US-A1- 2004 010 180
- US-A1- 2004 015 043

## Beschreibung

Die vorliegende Erfindung betrifft eine Vorrichtung zur epikardialen Unterstützung und/oder Übernahme der Herztätigkeit nach dem Oberbegriff des Anspruchs 1.

Eine Vorrichtung der vorgenannten Art ist bereits aus der Druckschrift DE 199 51 220 A1 bekannt. Es handelt sich dabei um ein wenig invasives, d. h. perkutan implantierbares System zur mechanischen Unterstützung und zum temporären Ersatz der Pumpenfunktion des Herzens. Die Vorrichtung wird nach Sondierung des Herzbeutels in zusammengefaltetem Zustand perkutan in den Herzbeutel eingebracht oder am Ende einer Operation in den Herzbeutel chirurgisch positioniert und dort mit der Doppelmembran um die rechte und linke Herzkammer gelegt. Dabei ist die Vorrichtung in deflatiertem Zustand so dünn, daß eine Kompression der Nachbarorgane vermieden wird. Nach der Implantation wird der Hohlraum der Doppelmembran über einen Verbindungsschlauch rhythmisch mit einem Fluid beaufschlagt, welches entweder ein Gas (Helium oder CO₂) oder eine geeignete Flüssigkeit sein kann. Durch dieses rhythmische Inflatieren und Deflatieren des Hohlraums der Doppelmembran und weil die äu-βere Membran im Gegensatz zur inneren Membran nicht dehnbar ist, kommt es zu einer Druckübertragung und Kompression des Herzens über die das Herz umschließende Doppelmembran. Dabei wird das Blut aus der rechten Herzkammer in die Pulmonalarterie und gleichzeitig aus der linken Kammer in die Aorta ausgetrieben oder bei vorhandener Pumpenfunktion des Herzens wird die systolische Auswurfarbeit des Herzmuskels unterstützt.

Derzeit wird allerdings noch bei jedem Patienten, bei dem ein chirurgischer Eingriff am Herzen vorgenommen wird, am Ende der Operation eine temporäre Schrittmachersonde epikardial aufgenäht. Für den Fall einer Asystopie (elektrischer Herzstillstand) bzw. einer Bradykardie (zu langsamer Herzschlag) während der kritischen postoperativen Phase kann das Herz mittels der beiden temporären Sonden über einen extern anzuschließenden Schrittmacher stimuliert werden. Nach fünf bis sieben Tagen werden die Sondenkabel herausgezogen, was ohne erneute Öffnung des Brustkorbes möglich ist. Allerdings wird es als Nachteil angesehen, daß die temporäre Schrittmachersonde jedes Mal aufgenäht werden muß.

Kommt es in der postoperativen Phase zu ventrikulären Rhythmusstörungen (Kammertachykardie oder Kammerflimmern), so muß das Herz mit einem Defibrillator wieder in den normalen Rhythmus überführt werden. Hierbei kommen zum einen von außen auf den Körper aufsetzbare Defibrillatoren zur Anwendung, von denen allerdings bekannt ist, daß die von dem Defibrillator in Form elektrischer Arbeit erzeugte Energie (200 bis 360 Ws) nur zu 10 bis 20 % direkt am Herzen wirkt, während der Hauptteil vom umliegenden Gewebe absorbiert wird. Zum anderen kommen implantierbare Defibrillatoren zu Anwendung, wobei in der Herzkammer Sonden plaziert werden, die in der Regel jahrelang im Patient verbleiben. Allerdings ist auch hierzu bekannt, daß in der Regel nur 30 bis 50 % der von einem implantierten Defibrillator erzeugten Energie (10 bis 34 Ws) am Herzen effektiv sind.

Aus der US 2003/0105481 A1 geht bereits eine Vorrichtung zur Unterstützung und/oder Übernahme der Herztätigkeit hervor, die einen kissenartigen Ballon aufweist. Der Ballon wird im Brustkorb neben dem Herzen außerhalb des Herzbeutels (Perikards) angeordnet. Auf der dem Herzen zugewandten Seite des Ballons befindet sich eine Sonden/Elektrodeneinheit zur perikardialen Ableitung eines EKG und/oder zur Signalübertragung und/oder -umsetzung eines externen Herzschrittmachers. Die Elektroden der Sonden Elektrodeneinheit liegen dabei unmittelbar auf dem Herzbeutel (Perikard) auf.

Die US 2001/0041821 A1 betrifft ein Verfahren und eine Vorrichtung in Form einer Doppelmembran, die in zusammengefaltetem Zustand perkutan in den Herzbeutel eingebracht wird. Die Doppelmembran ist manschettenartig aufgebaut, wobei bei der auf der dem Herzen zugewandten und am Herz anliegenden Innenseite der inneren Membran eine Sonden-/Elektrodeneinheit zur epikardialen Ableitung des EKGs und/oder Signalübertragung und -umsetzung eines externen Herzschrittmachers angeordnet ist.

Aufgabe der vorliegenden Erfindung ist es,eine Vorrichtung zur epikardialen Unterstützung und/oder Übernahme der Herztätigkeit zur Verfügung zu stellen, bei der eine vereinfachte postoperative Betreuung möglich ist.

Diese Aufgabe wird bei einer Vorrichtung zur epikardialen Unterstützung der Herztätigkeit der eingangs genannten Art erfindungsgemäß dadurch gelöst, dass auf der dem Herzen zugewandten Innenseite der inneren Membran wenigstens eine Sonden-/Elektrodeneinheit zur epikardialen Ableitung des EKGs und/der die Signalübertragung und -umsetzung eines externen Herzschrittmachers und Drucksensoren an der inneren Membran zum Messen des systolischen und des diastolischen Blutdrucks angeordnet sind.

Hierbei ist unter dem Begriff "Sonde" ein Messfühler für Herzströme oder Herzdrücke zu verstehen, und unter dem Begriff "Elektroden" elektrische Verbindungselemente zur Übertragung eines elektrischen Reizes oder Pulses auf das Herz.

Die Vorteile der erfindungsgemäßen Vorrichtung liegen insbesondere darin, dass das epikardiale EKG über die Sonden-/Elektrodeneinheit(en) abgeleitet und zur Triggerung, Ladekontrolle der Vorrichtung im Herzbeutel und als Funktionskontrolle benutzt werden. Dabei beinhaltet die Funktionskontrolle das Auslösen eines Alarms bei Herzrhythmusstörungen bis hin zur automatischen Aktivierung des Herzschrittmachers bzw. einer defibrillierenden Tätigkeit der Sonden-/Elektrodeneinheit(en). Damit stellt die Vorrichtung eine hervorragende Alternative zur konventionellen Triggerung über ein extern abgeleitetes EKG dar. Die epikardialen Sensoren der erfindungsgemäßen Vorrichtung sollen darüber hinaus über die Signale der Sonden-/Elektrodeneinheit(en) Daten über die Leistung des Herzens selbst übermitteln. So kann in der postoperativen Phase die Erholung des Patienten ("Weaning") überwacht, die Indikation zur Augmentation signalisiert und der Zeitpunkt für ein Entfernen der Vorrichtung angezeigt werden. Des weiteren ist selbstverständlich ein automatisches Umschalten von "Standby" (keine Unterstützung des Herzens) zu getriggerter Augmentation (Herz pumpt zum Teil selbst) bis hin zu komplettem Ersatz der Herztätigkeit (Herzstillstand) möglich.

Vorteilhafte Weiterbildungen der Erfindung sind in den Unteransprüchen 2 bis 8 angegeben.

Bevorzugterweise ist jedem der beiden Ventrikel wenigstens eine Sonden-/Elektrodeneinheit zugeordnet, was die erfindungsgemäße Vorrichtung auch bei einer beidseitigen Herzinsuffizienz einsetzbar macht.

Um eine Verbindung der Sonden-/Elektrodeneinheit(en) mit außerhalb des Patienten befindlichen Geräten zu ermöglichen, sind vorzugsweise Signalleitungen vorgesehen, die auf der Innenseite der inneren Membran der Doppelmembran geführt sind.

Vorzugsweise sind an der inneren Membran Drucksensoren zum Messen des systolischen und des diastolischen Blutdrucks angeordnet, mit denen die erfindungsgemäße Vorrichtung beispielsweise bei vorhandener Pumpfunktion des Herzens mit der Herzaktion synchronisierbar ist.

Auch für die Drucksensoren ist es von Vorteil, wenn die Signalübertragung der Drucksensoren zu einem externen Gerät über Signalleitungen erfolgt, die auf der Innenseite der inneren Membran geführt sind.

Vorzugsweise laufen die Signalleitungen der Sonden-/Elektrodeneinheit(en) und der Drucksensoren in einen gemeinsamen Anschlussstecker, der an ein entsprechendes Terminal einer externen Steuer- oder Antriebseinheit anschließbar ist.

Es ist vorgesehen, dass die Doppelmembran faltbar und die Sonden-/Elektrodeneinheit(en) sowie die Drucksensoren derart ausgebildet sind, dass sowohl die Implantation als auch deren Explantation durch eine perkutane Kanülierung erfolgen kann. Insofern ist eine Eröffnung des Brustkorbes weder bei der Operation noch postoperativ erforderlich.

Da nach koronaren Bypass-Operationen eine externe Kompression der epikardialen Gefäße nicht wünschenswert ist, weist die Doppelmembran im Bereich der großen Herzkranzgefäße vorzugsweise variable Aussparungen auf. Eine solche Doppelmembran kann entweder individuell auf den Patienten angepasst angefertigt oder aber, wie gemäß einer weiteren vorteilhaften Weiterbildung vorgesehen, mittels verschiebbarer Abstützungen an die besonderen Erfordernisse eines Patientenherzens angepasst werden. Dabei können die variablen Aussparungen durch faltbare, flexible Stege oder Halbschläuche durch mechanische Manipulation des Chirurgen in die gewünschte Position gebracht werden. Diese faltbaren, flexiblen Stege oder Halbschläuche können während des Pumpbetriebes entweder durch selbsthaftende Eigenschaften, Anwendung eines Gewebe-Klebstoffes, durch eine Halteschiene, oder durch Rillen innerhalb der Doppelmembran, welches bestimmte Positionen vorgeben, in der gewünschten Position gehalten werden.

Im folgenden wird ein Ausführungsbeispiel der Erfindung anhand einer Zeichnung näher erläutert.

Es zeigen:
- Figur 1:: eine schematische Darstellung der erfindungsgemäßen Vorrichtung;
- Figur 2:: eine schematische Darstellung eines Patienten mit einer implantierten erfindungsgemäßen Vorrichtung und einer externen Steuereinheit;
- Figur 3:: einen eröffneten Patientenoberkörper mit implantierter erfindungsgemäßer Vorrichtung; und
- Figur 4:: die Darstellung eines menschlichen Herzens mit aufgesetzter Vorrichtung und Aussparungen.

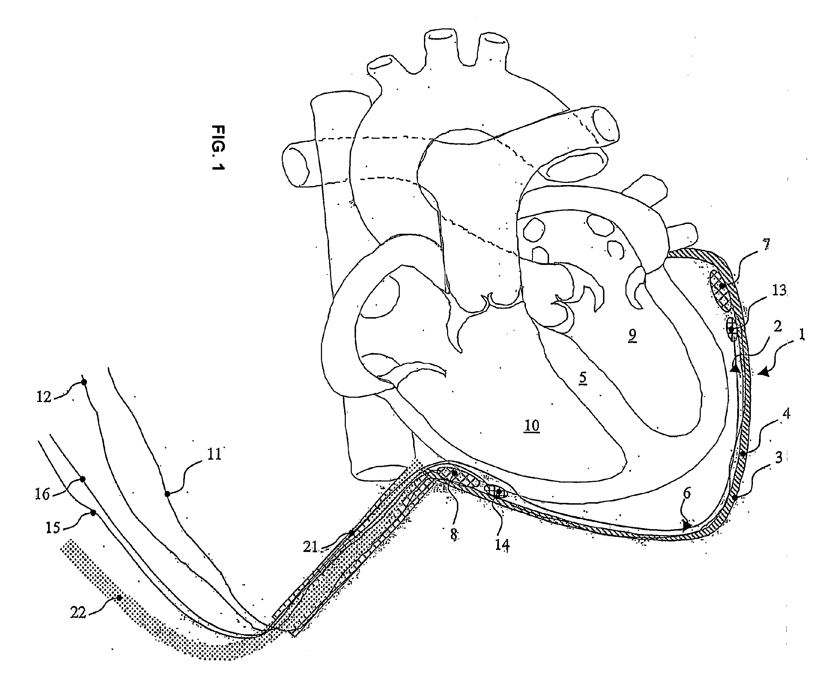

Figur 1 zeigt eine schematische Darstellung einer Vorrichtung zur epikardialen Unterstützung und/oder Übernahme der Herztätigkeit, mit einer Doppelmembran 1, die eine elastische innere Membran 2 und eine nicht dehnbare äußere Membran 3 sowie einen dazwischen gebildeten, geschlossenen und mittels eines Fluids inflatierbaren und deflatierbaren Hohlraum 4 aufweist. Auf der dem Herzen 5 zugewandten Innenseite 6 der inneren Membran 2 sind jedem der beiden Ventrikel 9, 10 zugeordnete Sonden-/Elektrodeneinheiten 7, 8 zur epikardialen Ableitung des EKGs und/oder zur Signalübertragung und - umsetzung eines externen Herzschrittmachers angeordnet. Diese Sonden-/Elektrodeneinheiten 7, 8 sind mittels Signalleitungen 11, 12, die auf der Innenseite 6 der inneren Membran 2 geführt sind, mit einem in dieser Figur nicht dargestellten externen Steuer- oder Antriebsgerät verbunden.

Des weiteren sind auf der Innenseite 6 der inneren Membran 2 Drucksensoren 13, 14 zum Messen des systolischen und diastolischen Blutdrucks angeordnet, die ebenfalls über auf der Innenseite 6 der inneren Membran 2 geführte Signalleitungen 15, 16 mit der hier nicht dargestellten externen Steuereinheit verbunden sind. Diese Signalleitungen 11, 12 und 15, 16 sowie ein Fluidschlauch 22 zum Inflatieren bzw. Deflatieren des Hohlraums 4 verlaufen im implantierten Zustand durch eine Einführschleuse 21 durch die Haut neben dem Brustbein des Patienten.

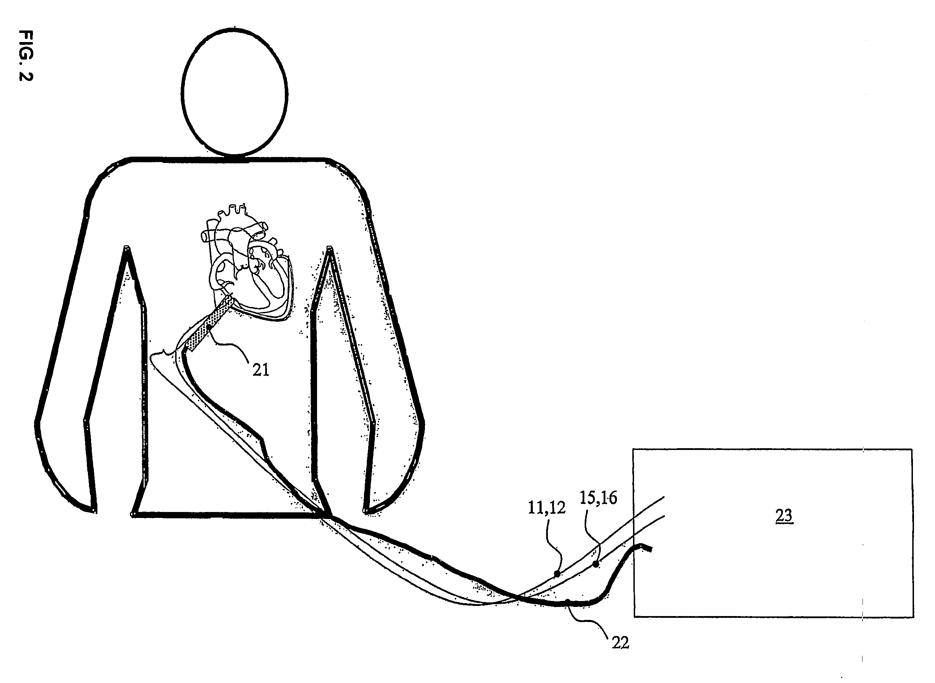

Figur 2 zeigt die schematische Darstellung der Lage einer implantierten Doppelmembran 1 in einem stilisiert dargestellten Patientenoberkörper sowie eine externe Steuer- und Antriebseinheit 23, welcher die Signalleitungen 11, 12 und 15, 16 sowie der Fluidschlauch 22 über die Einführschleuse 21 zugeführt werden.

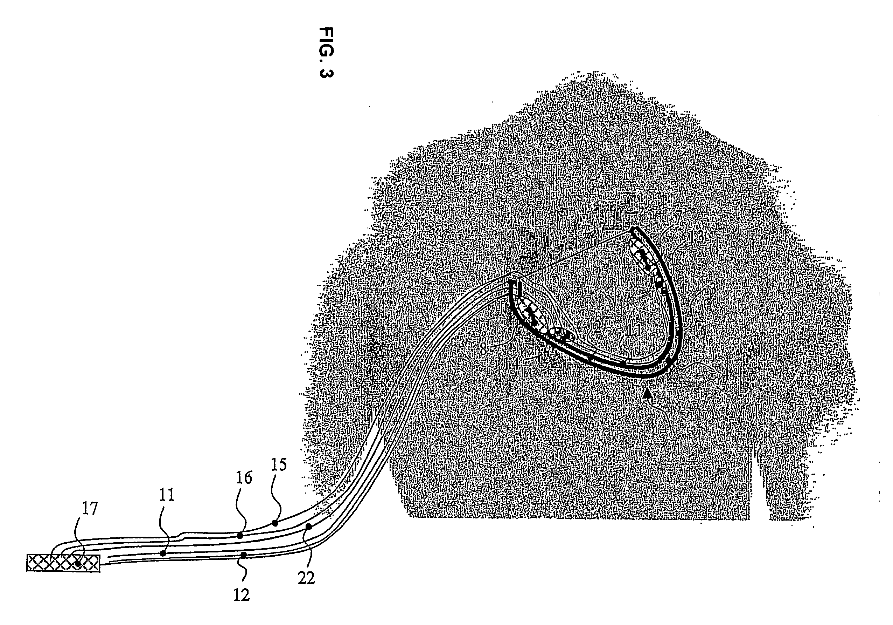

Figur 3 zeigt einen eröffneten Patientenoberkörper mit einer schematisch dargestellten und hineinprojizierten Doppelmembran 1 der vorliegenden Erfindung. Die Funktionselemente 7, 8 und 13, 14 sowie der Hohlraum 4 sind mittels des Fluidschlauchs 22 (hier nur durch eine Linie schematisch dargestellt) und mittels der Signalleitungen 11, 12 und 15, 16 an einen gemeinsamen Anschlussstecker 17 angeschlossen, mit dem die Signalleitungen und der Fluidschlauch wieder in einfacher Weise an die (in dieser Figur nicht dargestellte) externe Steuer- und Antriebseinheit 23 angeschlossen werden können.

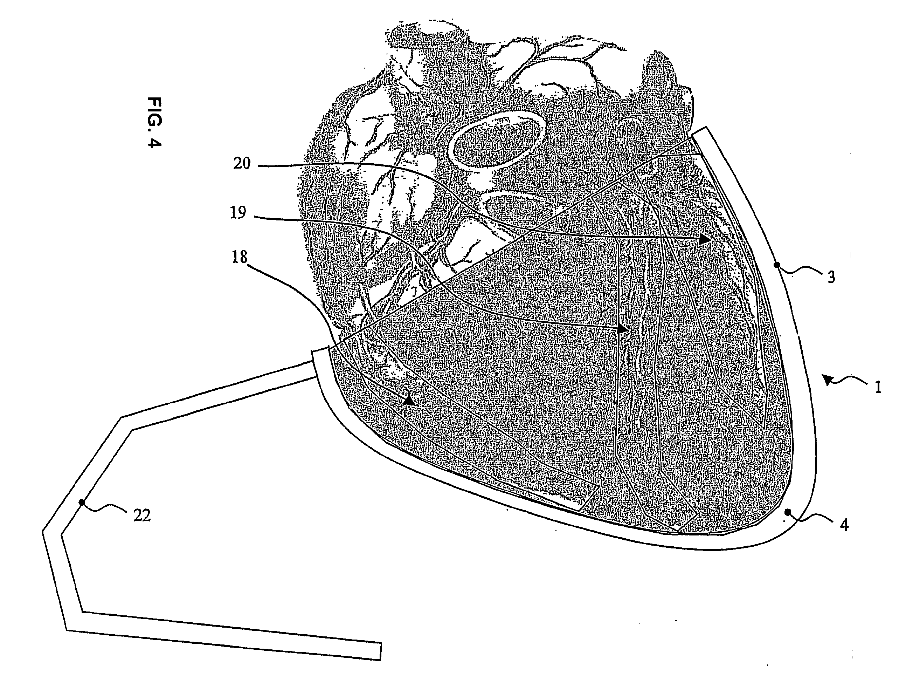

Figur 4 zeigt ein Patientenherz mit einer schematisch darum gelegten Doppelmembran 1, deren Hohlraum 4 wiederum über den Fluidschlauch 22 inflatierbar und deflatierbar ist. Dieses Ausführungsbeispiel der Doppelmembran 1 weist im Bereich der großen Herzkranzgefäße variable Aussparungen 18, 19, 20 auf, um eine externe Kompression der epikardialen Gefäße zu vermeiden. Diese Aussparungen 18, 19, 20 sind durch verschiebbare Abstützungen, die hier nicht dargestellt sind, an die Erfordernisse eines Patientenherzens anpassbar.

## Patentansprüche

1. Vorrichtung zur epikardialen Unterstützung und/oder Übernahme der Herztätigkeit, mit einer Doppelmembran (1), die eine elastische innere Membran (2) und eine nicht dehnbare äußere Membran (3) sowie einen dazwischen gebildeten, geschlossenen und mittels eines Fluides inflatierbaren und deflatierbaren Hohlraum (4) aufweist,
**dadurch gekennzeichnet, dass**
auf der dem Herzen (5) zugewandten Innenseite (6) der inneren Membran (2) wenigstens eine Sonden-/Elektrodeneinheit (7, 8) zur epikardialen Ableitung des EKGs und/der die Signalübertragung und -umsetzung eines externen Herzschrittmachers und Drucksensoren (13, 14) an der inneren Membran (2) zum Messen des systolischen und des diastolischen Blutdrucks angeordnet sind.

2. Vorrichtung nach Anspruch 1,
**dadurch gekennzeichnet, dass**
jedem der beiden Ventrikel (9, 10) wenigstens eine Sonden-/Elektrodeneinheit (7, 8) zugeordnet ist.

3. Vorrichtung nach Anspruch 1 oder 2,
**gekennzeichnet durch**
Signalleitungen (11, 12) zu den Sonden-/Elektrodeneinheiten (7, 8), die auf der Innenseite (6) der inneren Membran (2) geführt sind.

4. Vorrichtung nach Anspruch 3,
**gekennzeichnet durch**
Signalleitungen (15, 16) zu den Drucksensoren (13, 14), die auf der Innenseite (6) der inneren Membran (2) geführt sind.

5. Vorrichtung nach einem der Ansprüche 3 bis 4,
**gekennzeichnet durch**
einen gemeinsamen Anschlussstecker (17) für die Signalleitungen (11, 12; 15, 16).

6. Vorrichtung nach einem der vorstehenden Ansprüche
**dadurch gekennzeichnet, dass**
die Doppelmembran (1) faltbar und die Sonden-/Elektrodeneinheiten (7, 8) sowie die Drucksensoren (13, 14) derart ausgebildet sind, dass sowohl deren Implantation als auch deren Explantation durch eine perkutane Kanülierung erfolgen kann.

7. Vorrichtung nach einem der vorstehenden Ansprüche,
**gekennzeichnet durch**
variable Aussparungen (18, 19, 20) in der Doppelmembran (1) im Bereich der großen Herzkranzgefäße.

8. Vorrichtung nach Anspruch 7,
**gekennzeichnet durch**
verschiebbare Abstützungen, mittels derer die Aussparungen (18, 19, 20) an die Erfordernisse eines Patientenherzens anpassbar sind.

## Claims

1. Device for the epicardial support and/or taking over of cardiac activity, with a double membrane (1) which has an elastic inner membrane (2) and a non-stretchable outer membrane (3) and also a closed cavity (4) therebetween which is inflatable and deflatable by means of a fluid,
**characterized in that**
at least one probe/electrode unit (7, 8) for the epicardial recording of the ECG and/or for the signal transmission and conversion of an external cardiac pacemaker is arranged on the inner side (6) of the inner membrane (2) which faces the heart (5) and pressure sensors (13, 14) are arranged on the inner membrane (2) for measuring the systolic and the diastolic blood pressure.

2. Device according to Claim 1,
**characterized in that**
at least one probe/electrode unit (7, 8) is allocated to each of the two ventricles (9, 10).

3. Device according to Claim 1 or 2,
**characterized by**
signal leads (11, 12) to the probe/electrode units (7, 8), which are guided on the inner side (6) of the inner membrane (2).

4. Device according to Claim 3,
**characterized by**
signal leads (15, 16) to the pressure sensors (13, 14), which are guided on the inner side (6) of the inner membrane (2).

5. Device according to one of Claims 3 and 4,
**characterized by**
a common cable connector (17) for the signal leads (11, 12: 15, 16).

6. Device according to one of the preceding claims,
**characterized in that**
the double membrane (1) is foldable and the probe/electrode units (7, 8) and also the pressure sensors (13, 14) are designed in such a way that both their implantation and their explantation can be carried out by means of percutaneous cannulation.

7. Device according to one of the preceding claims,
**characterized by**
variable recesses (18, 19, 20) in the double membrane (1) in the region of the large coronary artery.

8. Device according Claim 7,
**characterized by**
displaceable supports, by means of which the recesses (18, 19, 20) can be adapted to the requirements of a patient's heart.

## Revendications

1. Dispositif pour l'assistance épicardique et/ou le rétablissement de l'activité cardiaque, comprenant une membrane double (1) qui présente une membrane intérieure élastique (2) et une membrane extérieure non extensible (3), ainsi qu'un espace creux (4) formé entre celles-ci, fermé et pouvant être gonflé et dégonflé au moyen d'un fluide,
**caractérisé en ce qu'**il est prévu, sur le côté intérieur (6) situé face au coeur (5) de la membrane intérieure (2), au moins une unité de sonde/d'électrode (7, 8) pour la dérivation épicardique de l'ECG et/ou la transmission et la conversion de signaux d'un stimulateur cardiaque externe, et sur la membrane intérieure (2), des capteurs de pression (13, 14) permettant de mesurer la tension artérielle systolique et diastolique.

2. Dispositif selon la revendication 1,
**caractérisé en ce qu'**au moins une unité de sonde/d'électrode (7, 8) est affectée à chacun des deux ventricules (9, 10).

3. Dispositif selon la revendication 1 ou 2,
**caractérisé par** des lignes de transmission de signaux (11, 12) vers les unités de sondes/d'électrodes (7, 8) qui s'étendent sur le côté intérieur (6) de la membrane intérieure (2).

4. Dispositif selon la revendication 3,
**caractérisé par** des lignes de transmission de signaux (15, 16) vers les capteurs de pression (13, 14) qui s'étendent sur le côté intérieur (6) de la membrane intérieure (2).

5. Dispositif selon l'une quelconque des revendications 3 à 4,
**caractérisé par** un connecteur adaptateur commun (17) pour les lignes de transmission de signaux (11, 12 ; 15, 16).

6. Dispositif selon l'une quelconque des revendications précédentes,
**caractérisé en ce que** la membrane double (1) est réalisée de façon repliable et les unités de sondes/d'électrodes (7, 8) ainsi que les capteurs de pression (13, 14) sont réalisées de telle sorte que leur implantation de même que leur explantation peut être effectuée au moyen d'une canule percutanée.

7. Dispositif selon l'une quelconque des revendications précédentes,
**caractérisé par** des évidements variables (18, 19, 20) dans la membrane double (1) dans la zone des grands vaisseaux coronaires.

8. Dispositif selon la revendication 7,
**caractérisé par** des supports coulissants au moyen desquels les évidements (18, 19, 20) peuvent être adaptés aux besoins du coeur d'un patient.
